(19) Europäisches Patentamt
European Patent Office
Office européen des brevets

(11) Veröffentlichungsnummer: **0 012 214**
**B1**

(12) # EUROPÄISCHE PATENTSCHRIFT

(45) Veröffentlichungstag der Patentschrift: **26.08.81**

(51) Int. Cl.³: **C 07 C 43/263**, C 07 C 29/09

(21) Anmeldenummer: **79104309.4**

(22) Anmeldetag: **05.11.79**

(54) **Verfahren zur Herstellung von 3-Phenoxy-benzylalkoholen.**

(30) Priorität: **18.11.78 DE 2850179**

(43) Veröffentlichungstag der Anmeldung: **25.06.80 Patentblatt 80/13**

(45) Bekanntmachung des Hinweises auf die Patenterteilung: **26.08.81 Patentblatt 81/34**

(84) Benannte Vertragsstaaten: **BE CH DE FR GB IT NL**

(56) Entgegenhaltungen:
**DE-A-2 402 457**
**DE-A1-2 707 232**
**GB-A-1 341 113**

(73) Patentinhaber: **BAYER AG, Zentralbereich Patente, Marken und Lizenzen, D-5090 Leverkusen 1, Bayerwerk (DE)**

(72) Erfinder: **Böhm, Siegfried, Dr., Silesiusstrasse 80, D-5000 Köln 80 (DE)**
Erfinder: **Klein, Alfons, Ing.-Grad., Virchowstrasse 9, D-4000 Düsseldorf (DE)**
Erfinder: **Wedemeyer, Karlfried, Dr., Bilharzstrasse 7, D-5000 Köln (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 012 214 B1

ACTORUM AG.

# Verfahren zur Herstellung von 3-Phenoxy-benzylalkoholen

Die Erfindung betrifft ein Verfahren zur Herstellung von 3-Phenoxy-benzylalkoholen aus 3-Phenoxy-benzylchloriden.

Die Umsetzung von 3-Phenoxy-benzylchlorid zum 3-Phenoxy-benzylalkohol ist bereits aus der DE-OS 2402457 bekannt. Danach wird das 3-Phenoxy-benzylchlorid zunächst mit Natriumacetat zum 3-Phenoxy-benylacetat umgesetzt und dann zum 3-Phenoxy-benzylalkohol verseift.

Es wurde ein Verfahren zur Herstellung von 3-Phenoxy-benzylalkoholen aus 3-Phenoxy-benzylchloriden gefunden, das dadurch gekennzeichnet ist, dass man 3-Phenoxy-benzylchloride der Formel

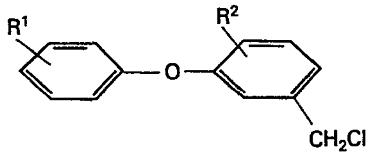

(I)

worin

$R^1$ und $R^2$ gleich oder verschieden sind für Wasserstoff oder Halogen stehen, im Temperaturbereich von 140 bis 210 °C unter Druck hydrolysiert.

Das erfindungsgemässe Verfahren kann beispielhaft anhand der folgenden Reaktionsgleichung erläutert werden:

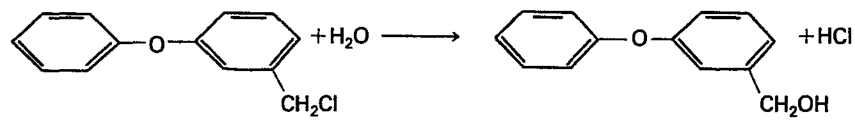


Halogene ($R^1$ und $R^2$) für das erfindungsgemässe Verfahren können Fluor, Chlor, Brom oder Jod, bevorzugt Fluor und Chlor, sein.

3-Phenoxy-benzylchloride für das erfindungsgemässe Verfahren sind bekannt (DE-OS 2704512). Sie können beispielsweise durch Seitenkettenchlorierung von 3-Phenoxy-toluolen in Gegenwart eines Radikalinitiators oder unter Belichtung hergestellt werden.

Die Hydrolyse nach dem erfindungsgemässen Verfahren kann in Gegenwart von wässrigen Basen durchgeführt werden.

Als wässrige Basen für das erfindungsgemässe Verfahren werden im allgemeinen wässrige Lösungen und/oder Suspensionen von Alkali- oder Erdalkali-Oxyden, Hydroxyden oder Carbonaten verwandt. Beispielsweise seien die folgenden Basen genannt: Natriumhydroxyd, Natriumcarbonat, Natriumhydrogencarbonat, Kaliumhydroxyd, Kaliumcarbonat, Kaliumhydrogencarbonat, Magnesiumoxyd, Magnesiumhydroxyd, Magnesiumcarbonat, Calciumoxyd, Calciumhydroxyd und Calciumcarbonat. Bevorzugte Basen für das erfindungsgemässe Verfahren sind Natriumhydroxyd, Natriumcarbonat, Calciumoxyd und MgO.

Die Alkali- bzw. Erdalkali-Verbindungen können einzeln oder im Gemisch verwendet werden. Es ist auch möglich, die Umsetzung mit einer dieser Verbindungen zu beginnen und im Verlauf der Reaktion eine andere zuzusetzen.

Für das erfindungsgemässe Verfahren kann die Menge der Base in weiten Grenzen schwanken. Im allgemeinen kann man 50 bis 150 Mol-%, vorzugsweise 75 bis 120 Mol-%, der dem Gehalt an Seitenkettenchlor im 3-Phenoxy-benzylchlorid, äquivalenten Mengen der Base einsetzen.

Zur Hydrolyse nach dem erfindungsgemässen Verfahren verwendet man im allgemeinen die 0,5 bis 10-fach, vorzugsweise die 1,5 bis 4-fache Menge Wasser, bezogen auf das eingesetzte Chlorierungsprodukt.

In einer bevorzugten Ausführungsform des erfindungsgemässen Verfahrens ist es möglich die Base nicht zu Beginn der Hydrolyse, sondern erst zu einem späteren Zeitpunkt umzusetzen. Beispielsweise gibt man nach 50 bis 85 Mol-% Umsatz, bezogen auf den Gehalt an Seitenkettenchlor im 3-Phenoxy-benzylchlorid, die Base hinzu.

Es ist aber auch möglich, die Hydrolyse ohne Zusatz einer Base durchzuführen.

Es ist auch möglich, die Hydrolyse in mehreren Stufen durchzuführen.

So kann man beispielsweise die Umsetzung nach etwa 40 bis 70% des Gesamtumsatzes abbrechen, die wässrige Phase entfernen und mit einer neuen wässrigen Phase zu Ende führen.

Die Hydrolyse nach dem erfindungsgemässen Verfahren wird im Temperaturbereich von 140 bis 210 °C, vorzugsweise von 150 bis 190 °C, durchgeführt. Die Reaktion wird zweckmässigerweise solange durchgeführt, bis sie im wesentlichen vollständig abgelaufen ist; dies ist im allgemeinen nach 2 bis 7 Stunden geschehen.

Das erfindungsgemässe Verfahren wird unter Druck durchgeführt. Im allgemeinen wird das erfindungsgemässe Verfahren im Druckbereich von 2 bis 100 bar, vorzugsweise von 3 bis 50 bar, durchgeführt. Das erfindungsgemässe Verfahren wird zweckmässigerweise in einem Druckgefäss, beispielsweise einem Autoklaven, durchgeführt. Es ist besonders bevorzugt, das erfindungsgemässe Verfahren unter dem Dampfdruck der Reaktanten im Autoklaven durchzuführen, der sich bei der erfindungsgemässen Temperatur einstellt.

Das erfindungsgemässe Verfahren kann beispielsweise wie folgt durchgeführt werden:

Das 3-Phenoxy-benzylchlorid, Wasser und gegebenenfalls die Base werden in einen Autoklaven gegeben und auf die Reaktionstemperatur erhitzt. Nach Beendigung der Reaktion kann das Reaktionsprodukt nach an sich bekannten Methoden aufgearbeitet werden. Beispielsweise trennt man

die wässrige Schicht von der organischen Phase ab und isoliert aus der organischen Phase den entstandenen 3-Phenoxy-benzylalkohol durch Destillation.

Nach dem erfindungsgemässen Verfahren können 3-Phenoxy-benzylalkohole der Formel

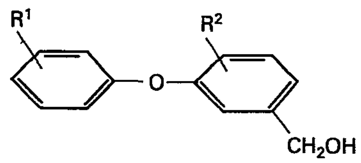

(II)

worin

$R_1$ und $R_2$ die obengenannten Bedeutung haben, hergestellt werden.

Die 3-Phenoxy-benzylalkohole können in Gegenwart von wässriger Schwefelsäure im Temperaturbereich von 50 bis 125 °C mit einer wässrigen Lösung eines Dichromats zu 3-Phenoxy-benzaldehyden oxydiert werden. Die 3-Phenoxy-benzaldehyde sind Zwischenprodukte für Pflanzenschutzmittel (Nachrichten aus Chemie, Technik und Laboratorium 26, 20 (1978)).

Die Herstellung der 3-Phenoxy-benzylalkohole nach dem erfindungsgemässen Verfahren erfolgt in einfacher Weise ohne Zwischenprodukte. Der 3-Phenoxy-benzylalkohol kann mit grosser Reinheit isoliert werden.

Es war nicht zu erwarten, dass unter den erfindungsgemässen Bedingungen die 3-Phenoxybenzylalkohole mit hohen Ausbeuten hergestellt werden können, da aus Ullmann, Enzyklopädie der Technischen Chemie, 4. Auflage, Band 8, Seite 437, bekannt ist, dass bei der Herstellung von Benzylalkohol aus Benzylchlorid durch Kochen mit wässrigen Lösungen von Alkali- bzw. Erdalkalihydroxyden oder auch deren Carbonaten als unvermeidbares Nebenprodukt bis zu 10% der Dibenzyläther anfallen. Verseifung unter Druck bei höheren Temperaturen und Erhöhung der Laugenkonzentration vermehren die Bildung des Dibenzyläthers, wobei dessen Anteil leicht bis auf 20% ansteigen kann.

Beispiel 1

623 g eines Chlorierungsproduktes folgender Zusammensetzung:

| | |
|---|---|
| Unbekannte Anteile | 1,6% |
| 3-Phenoxy-toluol | 32,4% |
| kernchlorierte Anteile | 1,2% |
| 3-Phenoxy-benzylchlorid | 53,4% |
| Unbekannte Anteile | 1,4% |
| 3-Phenoxy-benzalchlorid | 9,4% |
| Unbekannte Anteile | 0,5% |

Der Gehalt an Seitenkettenchlor beträgt 13,6%.

54 g Magnesiumoxid und

1240 g Wasser werden in einem Autoklaven 3 Stunden bei 180 °C gerührt. Nach dem Abkühlen trennt man von der wässrigen Schicht und destilliert die organische Phase nach Zugabe von

4 g 50%iger Natronlauge über eine 60 cm Laborfüllkörperkolonne:

Man erhält bei $Kp_{0,4}$ von 139 – 140 °C 263 g ein Phenoxy-benzylalkohol mit einem Reinheitsgrad von 99,5%.

Die Ausbeute beträgt 86,4% bezogen auf eingesetztes 3-Phenoxy-benzylchlorid.

Beispiel 2

Ein Chlorierungsprodukt folgender Zusammensetzung wird hydrolysiert:

| | |
|---|---|
| Unbekannte Anteile | 1,1% |
| 3-Phenoxy-toluol | 46,7% |
| kernchlorierte Anteile | 0,7% |
| 3-Phenoxy-benzalchlorid | 46,9% |
| Unbekannte Anteile | 1,0% |
| 3-Phenoxy-benzalchlorid | 3,6% |

Der Gehalt an Seitenkettenchlor beträgt 10,1%.

150 g dieses Chlorierungsgemisches

37,5 g 50%ige Natronlauge und

285 g Wasser werden in einem Autoklaven 3 Stunden bei 180 °C gerührt. Nach dem Abkühlen trennt man von der wässrigen Schicht und destilliert die organische Phase über eine Destillationsbrücke. Bei $Kp_{1,0}$ bis zu einer Temperatur von 190 °C erhält man 124,6 g Destillat. Es verbleibt ein Rückstand von 10,3 g.

Das Destillat ist wie folgt zusammengesetzt:

| | |
|---|---|
| Unbekannte Anteile | 0,4% |
| 3-Phenoxy-toluol | 48,9% |
| kernchlorierte Anteile | 0,6% |
| 3-Phenoxy-benzaldehyd | 4,6% |
| Unbekannte Anteile | 0,4% |
| 3-Phenoxy-benzylchlorid | 0,2% |
| 3-Phenoxy-benzylalkohol | 44,9% |

Bezogen auf das eingesetzte 3-Phenoxy-benzylchlorid beträgt die Ausbeute an 3-Phenoxy-benzylalkohol 87% des theoretischen Umsatzes.

Beispiel 3

150 g eines Chlorierungsproduktes der Zusammensetzung wie in Beispiel 2

13,3 g Calciumoxid und

300 g Wasser wurden 3 Stunden im Autoklaven bei 180 °C gerührt. Nach der Aufarbeitung, die analog derjenigen des Beispiels 1 erfolgt, erhält man ein Destillat von 124,2 g.

Es bleibt ein Rückstand von 6,5 g.

Zusammensetzung des Destillates:

| | |
|---|---|
| Unbekannte Anteile | 0,1% |
| 3-Phenoxy-toluol | 51,5% |
| kernchlorierte Anteile | 0,5% |
| 3-Phenoxy-benzaldehyd | 3,1% |
| Unbekannte Anteile | 0,1% |
| 3-Phenoxy-benzylchlorid | 0,1% |
| 3-Phenoxy-benzylalkohol | 44,6% |

Bezogen auf das eingesetzte 3-Phenoxy-benzylchlorid beträgt die Ausbeute an 3-Phenoxybenzylalkohol 86% des theoretischen Umsatzes.

Beispiel 4

100 g eines Chlorierungsgemisches mit folgender Zusammensetzung:

| | |
|---|---|
| Unbekannte Anteile | 1,6% |
| 3-Phenoxy-toluol | 19,1% |
| kernchlorierte Anteile | 1,2% |
| 3-Phenoxy-benzylchlorid | 64,1% |
| Unbekannte Anteile | 1,2% |
| 3-Phenoxy-benzalchlorid | 12,9% |

Der Gehalt an Seitenkettenchlor beträgt 15,9%.

27,3 g Natriumcarbonat und

250 g Wasser werden im Autoklaven 3 Stunden

bei 180 °C gerührt. Nach der Aufarbeitung, wie in Beispiel 2 beschrieben, erhält man 77,1 g Destillat und 6,7 g Rückstand.

Zusammensetzung des Destillats:

| | |
|---|---|
| Unbekannte Anteile | 0,9% |
| 3-Phenoxy-toluol | 29,7% |
| kernchlorierte Anteile | 1,2% |
| 3-Phenoxy-benzaldehyd | 11,3% |
| Unbekannte Anteile | 0,6% |
| 3-Phenoxy-benzylchlorid | 0,3% |
| 3-Phenoxy-benzylalkohol | 55,9% |

Bezogen auf das eingesetzte 3-Phenoxy-benzylchlorid beträgt die Ausbeute an 3-Phenoxybenzylalkohol 74% des theoretischen Umsatzes.

Beispiel 5

170 g eines Chlorierungsgemisches mit folgender Zusammensetzung:

| | |
|---|---|
| Unbekannte Anteile | 0,7% |
| 3-Phenoxy-4-fluor-toluol | 28,9% |
| kernchlorierte Anteile | 0,3% |
| 3-Phenoxy-4-fluor-benzylchlorid | 63,3% |
| Unbekannte Anteile | 0,5% |
| 3-Phenoxy-4-fluor-benzalchlorid | 6,3% |

Der Gehalt an Seitenkettenchlor beträgt 12,4%

53 g 50%ige Natronlauge und

310 g Wasser werden im Autoklaven 3 Stunden bei 180 °C gerührt. Nach dem Abkühlen wird die organische Phase in Toluol aufgenommen und von der wässrigen Schicht abgetrennt. Nach dem Abdestillieren des Toluols verbleiben

149 g Reaktionsprodukt mit folgender Zusammensetzung:

| | |
|---|---|
| Unbekannte Anteile | 0,2% |
| 3-Phenoxy-4-fluor-toluol | 39,3% |
| kernchlorierte Anteile | 0,6% |
| 3-Phenoxy-4-fluor-benzaldehyd | 2,3% |
| Unbekannte Anteile | 0,3% |
| 3-Phenoxy-4-fluor-benzylalkohol | 57,3% |

Bezogen auf das eingesetzte 3-Phenoxy-benzylchlorid beträgt die Ausbeute an 3-Phenoxybenzylalkohol 86% des theoretischen Umsatzes.

Beispiel 6

150 g chloriertes Material mit folgender Zusammensetzung:

| | |
|---|---|
| Unbekannte Anteile | 0,9% |
| 3-Phenoxy-toluol | 17,9% |
| kernchlorierte Anteile | 2,1% |
| 3-Phenoxy-benzylchlorid | 61,3% |
| Unbekannte Anteile | 1,3% |
| 3-Phenoxy-benzalchlorid | 16,8% |
| Unbekannte Anteile | 2,0% |

Der Gehalt an Seitenkettenchlor beträgt 15,9%.

480 g Wasser werden in einem Autoklaven 3 Stunden bei 160 °C gerührt. Man kühlt ab, trennt von der wässrigen Schicht und rührt die organische Phase nach Zugabe von weiteren

480 g Wasser nochmals 3 Stunden bei 160 °C.

Man erhält nach dem Abtrennen der wässrigen Schicht 125 g einer organischen Phase mit folgender Zusammensetzung:

| | |
|---|---|
| Unbekannte Anteile | 0,8% |
| 3-Phenoxy-toluol | 21,2% |
| kernchlorierte Anteile | 2,6% |
| 3-Phenoxy-benzaldehyd | 12,3% |
| 3-Phenoxy-benzylchlorid | 0,9% |
| Unbekannte Anteile | 1,8% |
| 3-Phenoxy-benzylalkohol | 60,1% |
| Unbekannte Anteile | 0,3% |

Bezogen auf das eingesetzte 3-Phenoxy-benzylchlorid beträgt die Ausbeute an 3-Phenoxybenzylalkohol 89% des theoretischen Umsatzes.

Beispiel 7

120 g chloriertes Material von der Zusammensetzung wie im Beispiel 6.

360 g Wasser werden in einem Autoklaven bei 170 °C insgesamt 7 Stunden gerührt.

Nach der 1. Stunde pumpt man 66 ml,

nach der 2. Stunde 35 ml

nach der 4. Stunde 35 ml und

nach der 6. Stunde 30 ml einer 10%igen Natronlauge zu.

Nach dem Abkühlen trennt man die wässrige Schicht ab und destilliert sie wie in Beispiel 2

beschrieben. Man erhält 98 g Destillat. Es verbleibt ein Rückstand von 5 g.

Das Destillat ist wie folgt zusammengesetzt:

| | |
|---|---|
| Unbekannte Anteile | 0,9% |
| 3-Phenoxy-toluol | 19,6% |
| kernchlorierte Anteile | 2,3% |
| 3-Phenoxy-benzaldehyd | 14.0% |
| 3-Phenoxy-benzylchlorid | 1,1% |
| Unbekannte Anteile | 1,3% |
| 3-Phenoxy-benzylalkohol | 60,3% |
| Unbekannte Anteile | 0,4% |

Bezogen auf eingesetztes 3-Phenoxy-benzylchlorid beträgt die Ausbeute an 3-Phenoxy-benzylalkohol 86% des theoretischen Umsatzes.

Beispiel 8

150 g chloriertes Material von der Zusammensetzung wie in Beispiel 6

300 g Wasser werden in einem Autoklaven bei 160 °C insgesamt 7 Stunden gerührt. Nach der 1. Stunde pumpt man 45 ml, nach der 2. Stunde 24 ml und nach der 6. Stunde 22 ml 20%ige Natronlauge zu. Nach dem Abkühlen trennt man die wässrige Schicht ab, versetzt die organische Phase mit weiteren

300 g Wasser und rührt nochmals 4 Stunden bei 160 °C. Die abgetrennte organische Phase wird destilliert. Man erhält 121 g Destillat. Es verbleibt ein Rückstand von 7,3 g.

Das Destillat ist wie folgt zusammengesetzt:

| | |
|---|---|
| Unbekannte Anteile | 0,7% |
| 3-Phenoxy-toluol | 21,0% |
| kernchlorierte Anteile | 2,8% |
| 3-Phenoxy-benzaldehyd | 13,3% |
| 3-Phenoxy-benylchlorid | 0,3% |
| Unbekannte Anteile | 1,6% |
| 3-Phenoxy-benzylalkohol | 59,8% |
| Unbekannte Anteile | 0,5% |

Bezogen auf eingesetztes 3-Phenoxy-benzylchlorid beträgt die Ausbeute an 3-Phenoxy-benzylalkahol 86% des theoretischen Umsatzes.

**Patentansprüche:**

1) Verfahren zur Herstellung von 3-Phenoxy-benzylalkoholen aus 3-Phenoxy-benzylchloriden, dadurch gekennzeichnet, dass man 3-Phenoxy-benzylchloride der Formel

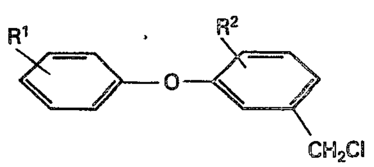

worin

$R^1$ und $R^2$ gleich oder verschieden sind und für Wasserstoff oder Halogen stehen, im Temperaturbereich von 140 bis 210 °C unter Druck hydrolysiert.

2) Verfahren nach Anspruch 1, dadurch gekennzeichnet, dass man die Umsetzung im Temperaturbereich von 150 bis 190 °C durchführt.

3) Verfahren nach den Ansprüchen 1 und 2, dadurch gekennzeichnet, dass man die Umsetzung im Druckbereich von 2 bis 100 bar durchführt.

4) Verfahren nach den Ansprüchen 1 bis 3, dadurch gekennzeichnet, dass man die Hydrolyse in Gegenwart einer Base durchführt.

5) Verfahren nach den Ansprüchen 1 bis 4, dadurch gekennzeichnet, dass man als Base eine wässrige Lösung und/oder Suspension von Alkali- oder Erdalkalioxyden, Hydroxyden oder Carbonaten verwendet.

6) Verfahren nach den Ansprüchen 1 bis 5, dadurch gekennzeichnet, dass man als wässrige Base wässriges Natriumhydroxyd, Natriumcarbonat, Calciumoxid und/oder Magnesiumoxid einsetzt.

7) Verfahren nach den Ansprüchen 1 bis 6, dadurch gekennzeichnet, dass man die Base zu einem späteren Zeitpunkt der Hydrolyse zusetzt.

8) Verfahren nach den Ansprüchen 1 bis 7, dadurch gekennzeichnet, dass man die Hydrolyse in mehreren Stufen durchführt.

9) Verfahren nach den Ansprüchen 1 bis 8, dadurch gekennzeichnet, dass man 50 bis 150 Mol-% der dem Gehalt an Seitenkettenchlor im 3-Phenoxy-benzylchlorid äquivalenten Menge der wässrigen Base verwendet.

10) Verfahren nach den Ansprüchen 1 bis 9, dadurch gekennzeichnet, dass man 0,5 bis 10 Mol Wasser, bezogen auf 1 Mol des eingesetzten 3-Phenoxy-benzylchlorids einsetzt.

**Revendications**

1. Procédé de fabrication d'alcools 3-phénoxy-benzyliques à partir de chlorures de 3-phénoxy-benzyle, caractérisé en ce qu'on hydrolyse sous pression dans l'intervalle de température de 140 à 210 °C des chlorures de 3-phénoxy-benzyle de formule:

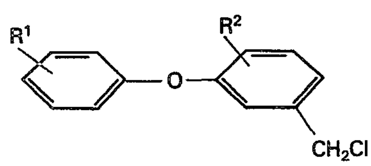

(I)

dans laquelle

$R^1$ et $R^2$ sont identiques ou différents e représentent de l'hydrogène ou de l'halogène.

2. Procédé selon la revendication 1, caractérisé en ce qu'on exécute la réaction dans l'intervalle de température de 150 à 190 °C.

3. Procédé selon l'une quelconque des revendications 1 et 2, caractérisé en ce qu'on exécute la réaction dans l'intervalle de pression de 2 à 100 bars.

4. Procédé selon l'une quelconque des revendications 1 à 3, caractérisé en ce qu'on exécute l'hydrolyse en présence d'une base.

5. Procédé selon l'une quelconque des revendications 1 à 4, caractérisé en ce qu'en tant que base on utilise une solution et/ou suspension aqueuse d'oxydes, hydroxydes ou carbonates alcalins ou alcalino-terreux.

6. Procédé selon l'une quelconque des revendications 1 à 5, caractérisé en ce qu'on utilise en tant que base aqueuse de l'hydroxyde de sodium, carbonate de sodium, oxyde de calcium et/ou oxyde de mangésium aqueux.

7. Procédé selon l'une quelconque des revendications 1 à 6, caractérisé en ce qu'on ajoute la base à un moment plus tardif de l'hydrolyse.

8. Procédé selon l'une quelconque des revendications 1 à 7, caractérisé en ce qu'on exécute l'hydrolyse en plusieurs stades.

9. Procédé selon l'une quelconque des revendications 1 à 8, caractérisé en ce qu'on utilise 50 à 150 moles % de la quantité de base aqueuse équivalente à la teneur en chlore sur la chaîne latérale dans le chlorure de 3-phénoxy-benzyle.

10. Procédé selon l'une quelconque des revendications 1 à 9, caractérisé en ce qu'on utilise 0,5 à 10 moles d'eau par mole du chlorure de 3-phénoxy-benzyle mis en jeu.

**Claims**

1. Process for the preparation of 3-phenoxy-benzyl alcohols from 3-phenoxy-benzyl chlorides characterised in that 3-phenoxy-benzyl chlorides of the formula

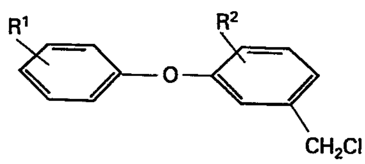

wherein

$R^1$ and $R^2$ are identical or different and represent hydrogen or halogen, are hydrolysed under pressure in a temperature range from 140 to 210 °C.

2. Process according to Claim, 1, characterised in that the conversion is carried out in a temperature range from 150 to 190 °C.

3. Process according to Claims 1 and 2, characterised in that the conversion is carried out in a pressure range from 2 to 100 bars.

4. Process according to Claims 1 to 3, characterised in that the hydrolysis is carried out in the presence of a base.

5. Process according to Claims 1 to 4, characterised in that an aqueous solution and/or suspension of alkali metal oxides or alkaline earth metal oxides, hydroxides or carbonates is used as the base.

6. Process according to Claims 1 to 5, characterised in that auqeous sodium hydroxide, sodium carbonate, calcium oxide and/or magnesium oxide is employed as the aqueous base.

7. Process according to Claims 1 to 6, characterised in that the base is added at a later point in time of the hydrolysis.

8. Process according to Claims 1 to 7, characterised in that the hydrolysis is carried out in several stages.

9. Process according to Claims 1 to 8, characterised in that 50 to 150 mol % of the amount of aqueous base equivalent to the content of side-chain chlorine in the 3-phenoxy-benzyl chloride are used.

10. Process according to Claims 1 to 9, characterised that 0,5 to 10 mols of water, relative to 1 mol of the 3-phenoxy-benzyl chloride employed, are used.